# EUROPEAN PATENT SPECIFICATION

(11) **EP 2 512 514 B1**
(45) Date of publication and mention of the grant of the patent: **05.11.2014**
(21) Application number: 10837714.4
(22) Date of filing: 14.12.2010
(51) Int. Cl.: A61K 45/00, A61P 25/02, C12N 5/10, C12N 15/09, G01N 33/68, G01N 33/50

(54) **SCREENING METHOD FOR IDENTIFYING COMPOUNDS FOR TREATING AMYOTROPHIC LATERAL SCLEROSIS**
SCREENINGVERFAHREN ZUR IDENTIFIZIERUNG VON VERBINDUNGEN ZUR BEHANDLUNG VON AMYOTROPHER LATERALSKLEROSE
PROCÉDÉ DE CRIBLAGE PERMETTANT D'IDENTIFIER DES COMPOSÉS POUR LE TRAITEMENT DE LA SCLÉROSE LATÉRALE AMYOTROPHIQUE

(30) Priority: 14.12.2009 US 286134 P
(43) Date of publication of application: 24.10.2012
(73) Proprietor: Kyoto University, Sakyo-ku Kyoto-shi Kyoto 606-8501 (JP)
(72) Inventor: INOUE, Haruhisa, Kyoto-shi Kyoto 606-8507 (JP); NAKATSUJI, Norio, Kyoto-shi Kyoto 606-8501 (JP); KITAOKA, Shiho, Kyoto-shi Kyoto 606-8507 (JP); TSUKITA, Kayoko, Kyoto-shi Kyoto 606-8507 (JP); TAKAHASHI, Ryosuke, Kyoto-shi Kyoto 606-8501 (JP); MURAKAMI, Gaku, Kyoto-shi Kyoto 606-8501 (JP); AMAGAI, Yuji, Kyoto-shi Kyoto 606-0805 (JP); AIBA, Kazuhiro, Kyoto-shi Kyoto 606-0805 (JP)
(74) Representative: Duckworth, Timothy John
(86) International application number: PCT/JP2010/072836
(87) International publication number: WO 2011/074690

(56) References cited:
- WO-A1-2006/105806
- WO-A1-2008/127974
- WO-A2-2004/000313
- WO-A2-2009/146098
- JP-A- 2008 542 200
- US-A1- 2005 202 488
- BROOM W J ET AL: "Two approaches to drug discovery in SOD1-mediated ALS", JOURNAL OF BIOMOLECULAR SCREENING, LARCHMONT, NY, US, vol. 11, no. 7, 1 October 2006 (2006-10-01), pages 729-735, XP008102024, ISSN: 1087-0571, DOI: 10.1177/1087057106290937
- DIMOS JOHN T ET AL: "Induced pluripotent stem cells generated from patients with ALS can be differentiated into motor neurons", SCIENCE, AMERICAN ASSOCIATION FOR THE ADVANCEMENT OF SCIENCE, US, vol. 321, no. 5893, 29 August 2008 (2008-08-29), pages 1218-1221, XP002552499, ISSN: 1095-9203, DOI: 10.1126/SCIENCE.1158799
- BERGERON C ET AL: "Copper/zinc superoxide dismutase mRNA levels are increased in sporadic amyotrophic lateral sclerosis motorneurons", BRAIN RESEARCH, ELSEVIER, AMSTERDAM, NL, vol. 659, no. 1-2, 3 October 1994 (1994-10-03), pages 272-276, XP024284611, ISSN: 0006-8993, DOI: 10.1016/0006-8993(94)90892-3 [retrieved on 1994-10-03]
- BATTISTINI S ET AL: "Severe familial ALS with a novel exon 4 mutation (L106F) in the SOD1 gene", JOURNAL OF NEUROLOGICAL SCIENCES, ELSEVIER SCIENTIFIC PUBLISHING CO, AMSTERDAM, NL, vol. 293, no. 1-2, 15 June 2010 (2010-06-15), pages 112-115, XP027119335, ISSN: 0022-510X [retrieved on 2010-04-10]
- BRUIJN,L.I. ET AL.: 'Unraveling the mechanisms involved in motor neuron degeneration in ALS' ANNU REV NEUROSCI vol. 27, 2004, pages 723 - 49
- IWAMOTO ET AL.: "Atorvastatin treatment attenuates motor neuron degeneration in wobbler mice.", AMYOTROPHIC LATERAL SCLEROSIS, vol. 10, 2009, pages 405-409,

### SEQUENCE LISTING

<110> Kyoto University
<120> PHARMACEUTICAL COMPOSITION FOR PREVENTION AND TREATMENT OF AMYOTROPHIC LATERAL SCLEROSIS
<130> 091651
<150> US 61/286,134
   <151> 2009-12-14
<160> 2
<170> PatentIn version 3.5
<210> 1
   <211> 981
   <212> DNA
   <213> Homo sapiens
<220>
   <221> CDS
   <222> (149).. (613)
<400> 1
   gtttggggcc agagtgggcg aggcgcggag gtctggccta taaagtagtc gcggagacgg 60
   ggtgctggtt tgcgtcgtag tctcctgcag cgtctggggt ttccgttgca gtcctcggaa 120
   ccaggacctc ggcgtggcct agcgagtt atg gcg acg aag gcc gtg tgc gtg 172
   Met Ala Thr Lys Ala Val Cys Val
   1 5
   ctg aag ggc gac ggc cca gtg cag ggc atc atc aat ttc gag cag aag 220
   Leu Lys Gly Asp Gly Pro Val Gln Gly Ile Ile Asn Phe Glu Gln Lys
   10 15 20
   gaa agt aat gga cca gtg aag gtg tgg gga agc att aaa gga ctg act 268
   Glu Ser Asn Gly Pro Val Lys Val Trp Gly Ser Ile Lys Gly Leu Thr
   25 30 35 40
   gaa ggc ctg cat gga ttc cat gtt cat gag ttt gga gat aat aca gca 316
   Glu Gly Leu His Gly Phe His Val His Glu Phe Gly Asp Asn Thr Ala
   45 50 55
   ggc tgt acc agt gca ggt cct cac ttt aat cct cta tcc aga aaa cac 364
   Gly Cys Thr Ser Ala Gly Pro His Phe Asn Pro Leu Ser Arg Lys His
   60 65 70
   ggt ggg cca aag gat gaa gag agg cat gtt gga gac ttg ggc aat gtg 412
   Gly Gly Pro Lys Asp Glu Glu Arg His Val Gly Asp Leu Gly Asn Val
   75 80 85
   act gct gac aaa gat ggt gtg gcc gat gtg tct att gaa gat tct gtg 460
   Thr Ala Asp Lys Asp Gly Val Ala Asp Val Ser Ile Glu Asp Ser Val
   90 95 100
   atc tca gtc tca gga gac cat tgc atc att ggc cgc aca ctg gtg gtc 508
   Ile Ser Val Ser Gly Asp His Cys Ile Ile Gly Arg Thr Leu Val Val
   105 110 115 120
   cat gaa aaa gca gat gac ttg ggc aaa ggt gga aat gaa gaa agt aca 556
   His Glu Lys Ala Asp Asp Leu Gly Lys Gly Gly Asn Glu Glu Ser Thr
   125 130 135
   aag aca gga aac gct gga agt cgt ttg gct tgt ggt gta att ggg atc 604
   Lys Thr Gly Asn Ala Gly Ser Arg Leu Ala Cys Gly Val Ile Gly Ile
   140 145 150
   gcc caa taa acattccctt ggatgtagtc tgaggcccct taactcatct 653
   Ala Gln
   gttatcctgc tagctgtaga aatgtatcct gataaacatt aaacactgta atcttaaaag 713
   tgtaattgtg tgactttttc agagttgctt taaagtacct gtagtgagaa actgatttat 773
   gatcacttgg aagatttgta tagttttata aaactcagtt aaaatgtctg tttcaatgac 833
   ctgtattttg ccagacttaa atcacagatg ggtattaaac ttgtcagaat ttctttgtca 893
   ttcaagcctg tgaataaaaa ccctgtatgg cacttattat gaggctatta aaagaatcca 953
   aattcaaact aaaaaaaaaa aaaaaaaa 981
<210> 2
   <211> 154
   <212> PRT
   <213> Homo sapiens
<400> 2
   Met Ala Thr Lys Ala Val Cys Val Leu Lys Gly Asp Gly Pro Val Gln
   1 5 10 15
   Gly Ile Ile Asn Phe Glu Gln Lys Glu Ser Asn Gly Pro Val Lys Val
   20 25 30
   Trp Gly Ser Ile Lys Gly Leu Thr Glu Gly Leu His Gly Phe His Val
   35 40 45
   His Glu Phe Gly Asp Asn Thr Ala Gly Cys Thr Ser Ala Gly Pro His
   50 55 60
   Phe Asn Pro Leu Ser Arg Lys His Gly Gly Pro Lys Asp Glu Glu Arg
   65 70 75 80
   His Val Gly Asp Leu Gly Asn Val Thr Ala Asp Lys Asp Gly Val Ala
   85 90 95
   Asp Val Ser Ile Glu Asp Ser Val Ile Ser Val Ser Gly Asp His Cys
   100 105 110
   Ile Ile Gly Arg Thr Leu Val Val His Glu Lys Ala Asp Asp Leu Gly
   115 120 125
   Lys Gly Gly Asn Glu Glu Ser Thr Lys Thr Gly Asn Ala Gly Ser Arg
   130 135 140
   Leu Ala Cys Gly Val Ile Gly Ile Ala Gln
   145 150

## Description

### Technical Field of the Invention

The present invention relates to a method of screening for a prophylactic and therapeutic drug for amyotrophic lateral sclerosis.

### Background of the Invention

Amyotrophic lateral sclerosis (hereinafter, ALS) is a motor neuron disease of poor prognosis, which develops at middle ages and thereafter and causes progressive paralysis of skeletal muscles. It is designated as a disease in the project for investigation and research into specific diseases sponsored by the Ministry of Health, Labor and Welfare of Japan. More than about 90% of cases of ALS are sporadic and the cause is unknown, whereas the remaining 10% are familial cases. To explain the causal factor in the latter cases, the gain-of-toxic function theory is likely wherein motor neuron death is caused by the cytotoxicity newly gained by mutated SOD1 (Cu/Zn superoxide dismutase) as a result of a point mutation of the SOD1 gene (Bruijn, L.I., et al., 2004. Annu. Rev. Neurosci. 27, 723-749).

The only currently commercially available therapeutic drug for ALS is riluzole (Rilutek^{™}, Aventis), a glutamate receptor antagonist possessing glutamate suppressing action (AU 666150 B2).

In recent years, mouse and human induced pluripotent stem cells (iPS cells) have been established one after another. Yamanaka et al. induced iPS cells by transferring into mouse fibroblasts the Oct3/4, Sox2, Klf4 and c-Myc genes, and forcing the fibroblasts to express the genes [WO 2007/069666 A1; Takahashi, K. and Yamanaka, S., Cell, 126: 663-676 (2006)]. Thereafter, it was revealed that iPS cells could also be produced with three of the factors other than the c-Myc gene [Nakagawa, M. et al., Nat. Biotechnol., 26: 101-106 (2008)]. Furthermore, Yamanaka et al. succeeded in establishing iPS cells by transferring into human skin fibroblasts the same four genes as those used in the mouse [WO 2007/069666 A1; Takahashi, K. et al., Cell, 131: 861-872 (2007)]. Meanwhile, Thomson and his colleagues produced human iPS cells using Nanog and Lin28 in place of Klf4 and c-Myc [WO 2008/118820 A2; Yu, J. et al., Science, 318: 1917-1920 (2007)]. Because the iPS cells thus obtained can be differentiated into cells of various tissues after being generated using cells derived from the patient to be treated, they are thought to enable reproduction of the pathologic condition in vitro. In fact, the above-described method was successfully applied to generate iPS cells from ALS patient and differentiate into neurons [Dimos JT, et al., Science, 2008, 321(5893):1218-21].

However, using a neuron derived from an iPS cell, no contributory therapeutic drugs for ALS have been discovered to date.

### Summary of the Invention

It is an object of the present invention to provide a screening method for a drug that is useful in preventing and treating ALS. Accordingly, the present inventors established iPS cells from fibroblasts of an ALS patient, and differentiated them into astrocytes. Here, with a focus on the amount of SOD1 expressed, the astrocytes thus obtained and a test compound were brought into contact with each other, and a compound that reduces the amount expressed was screened for. As a result, an HMG-CoA reductase inhibitor was found to reduce the amount of SOD1 expressed.

Judging from these results, the present inventors found that a pharmaceutical composition for prevention and treatment of ALS can be screened for by determining the amount of SOD1 expressed using an astrocyte differentiated from an iPS cell derived from an ALS patient, and that an HMG-CoA reductase inhibitor is useful for preventing and treating ALS.

Accordingly, the present invention provides the following:
[1] A method of screening for a prophylactic and therapeutic drug for amyotrophic lateral sclerosis (ALS), comprising the steps of:
   (1) differentiating an astrocyte from an induced pluripotent stem (iPS) cell having a mutation of Cu/Zn superoxide dismutase (SOD1) that correlates with ALS by (a) forming a neurosphere from the iPS cell, and (b) culturing the neurosphere in a medium in a coated culture dish,
   (2) bringing into contact with each other the astrocyte and a test compound,
   (3) measuring the amount of SOD1 expressed in the astrocyte, and
   (4) selecting a test compound that reduces the amount of SOD1 expressed, compared with a control not brought into contact with the test compound.
[2] The screening method according to [1], wherein the iPS cell is an iPS cell having a mutation from leucine to valine at position 106 of SOD1.
[3] The screening method according to [2], wherein the medium for culturing the neurosphere contains a leukemia inhibitory factor (LIF) and a bone morphogenetic protein (BMP).

According to the present invention, it is possible to screen for a prophylactic and therapeutic drug for ALS using an astrocyte differentiated from an iPS cell. The present invention is therefore highly useful in identifying compounds for preventing and treating ALS, and in developing a pharmaceutical composition for prevention and treatment of the disease.

### Brief Description of the Drawings

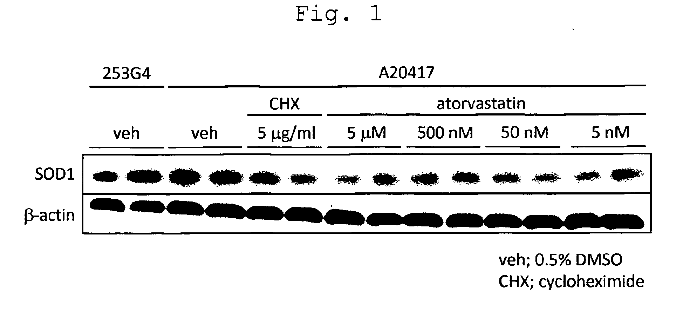

Fig. 1 shows results of amount of SOD1 protein in the case of the addition of each drug to iPS cell-derived astrocytes, wherein "veh" shows the results obtained with the addition of DMSO alone, "CHX" with the addition of cycloheximide, and "Atorvastatin" with the addition of the indicated concentrations of Atorvastatin Calcium Salt. Each experiment was performed in duplicate; all results obtained are shown.

### Detailed Description of the Invention

The present invention provides a method of screening for a compound that reduces the expression of SOD1 in an astrocyte differentiated from an iPS cell derived from an ALS patient having a mutation in SOD1. Also disclosed herein is a pharmaceutical composition for prevention or treatment of ALS containing an HMG-CoA reductase inhibitor identified by the screening method.

### I. Production of iPS cells

An iPS cell is an artificial stem cell derived from somatic cell, which has nearly the same characteristics as those of ES cells, for example, differentiation pluripotency and the potential for proliferation by self-renewal, and that can be prepared by transferring a certain nuclear reprogramming substance, in the form of nucleic acid or protein, to a somatic cell [K. Takahashi and S. Yamanaka (2006) Cell, 126: 663-676; K. Takahashi et al. (2007) Cell, 131: 861-872; J. Yu et al. (2007) Science, 318: 1917-1920; M. Nakagawa et al. (2008) Nat. Biotechnol., 26: 101-106; WO 2007/069666]. The nuclear reprogramming substance may be any gene specifically expressed in ES cells, or a gene that plays a key role in the maintenance of the undifferentiated state of ES cells, or a gene product thereof. Examples include Oct3/4, Klf4, Klf1, Klf2, Klf5, Sox2, Sox1, Sox3, Sox15, Sox17, Sox18, c-Myc, L-Myc, N-Myc, TERT, SV40 Large T antigen, HPV16 E6, HPV16 E7, Bmil, Lin28, Lin28b, Nanog, Esrrb and Esrrg. These reprogramming substances may be used in combination when establishing iPS cells. For example, a combination comprising at least one, two or three of these reprogramming substances may be used, with preference given to a combination comprising four.

Information on the nucleotide sequences of the mouse and human cDNAs of the above-described nuclear reprogramming substances and the amino acid sequences of the proteins encoded thereby is available with reference to the NCBI accession numbers shown in WO 2007/069666. Information on the mouse and human cDNA sequences and amino acid sequences of L-Myc, Lin28, Lin28b, Esrrb and Esrrg is available with reference to the NCBI accession numbers shown below. Those skilled in the art are easily able to prepare a desired nuclear reprogramming substance by a conventional method on the basis of the information on the cDNA sequence or amino acid sequence thereof.

| Name of gene | Mouse | Human |
|---|---|---|
| L-Myc | NM_008506 | NM_001033081 |
| Lin28 | NM_145833 | NM_024674 |
| Lin28b | NM_001031772 | NM_001004317 |
| Esrrb | NM_011934 | NM_004452 |
| Esrrg | NM_011935 | NM_001438 |

These nuclear reprogramming substances may be transferred to somatic cells in the form of a protein by means of, for example, lipofection, binding to cell membrane permeable peptides, and microinjection, or may be transferred to somatic cells in the form of DNA by means of, for example, vectors such as viruses, plasmids, and artificial chromosomes, as well as lipofection, liposomes, and microinjection. Examples of viral vectors include retrovirus vectors, lentivirus vectors (both Cell, 126, pp. 663-676, 2006; Cell, 131, pp. 861-872, 2007; Science, 318, pp. 1917-1920, 2007), adenovirus vectors (Science, 322, 945-949, 2008), adeno-associated virus vectors, Sendai virus vectors (Proc. Jpn. Acad. Ser. B. Phys. Biol. Sci. 85, 348-62, 2009) and the like. Artificial chromosomal vectors include, for example, human artificial chromosome (HAC), yeast artificial chromosome (YAC), bacterial artificial chromosome (BAC, PAC) and the like. Plasmids for mammalian cells can be used (Science, 322:949-953, 2008). The vector can contain a regulatory sequence such as a promoter, enhancer, ribosome-binding sequence, terminator, or polyadenylation site to allow a nuclear reprogramming substance to be expressed, and can further contain, as required, a drug resistance gene (e.g., kanamycin resistance gene, ampicillin resistance gene, puromycin resistance gene and the like), a selection marker sequence such as the thymidine kinase gene or diphtheria toxin gene, a reporter gene sequence such as of green fluorescent protein (GFP), β glucuronidase (GUS) or FLAG, and the like. The vector may have a loxP sequence placed at both ends of the gene that encodes the nuclear reprogramming substance or of a promoter and the gene connected thereto, to enable resection thereof, after being transferred to somatic cells. The vector may also contain the EBNA-1 and oriP sequences or the Large T and SV40ori sequences to allow the vector to be replicated and occur episomally even without being incorporated in the chromosome.

To increase iPS cell induction efficiency in nuclear reprogramming, in addition to the above-described factors, for example, histone deacetylase (HDAC) inhibitors [e.g., valproic acid (VPA) (Nat. Biotechnol., 26(7) : 795-797 (2008)], low-molecular inhibitors such as trichostatin A, sodium butyrate, MC 1293, and M344, nucleic acid-based expression inhibitors such as siRNAs and shRNAs against HDAC (e.g., HDAC1 siRNA Smartpool^{®} (Millipore), HuSH 29mer shRNA constructs against HDAC1 (OriGene) and the like), and the like], DNA methyltransferase inhibitors (e.g., 5'-azacytidine) [Nat. Biotechnol., 26(7): 795-797 (2008)], G9a histone methyltransferase inhibitors [e.g., low-molecular inhibitors such as BIX-01294 (Cell Stem Cell, 2: 525-528 (2008)], nucleic acid-based expression inhibitors such as siRNAs and shRNAs against G9a [e.g., G9a siRNA (human) (Santa Cruz Biotechnology) and the like) and the like], L-channel calcium agonists (e.g., Bayk8644) [Cell Stem Cell, 3, 568-574 (2008)], p53 inhibitors [e.g., siRNA and shRNA against p53 (Cell Stem Cell, 3, 475-479 (2008)), Wnt Signaling (e.g., soluble Wnt3a) [Cell Stem Cell, 3, 132-135 (2008)], cytokines such as LIF, bFGF etc., ALK5 inhibitors (e.g., SB431542) [Nat Methods, 6: 805-8 (2009)], a mitogen-activated protein kinase signaling inhibitor, a glycogen synthase kinase-3 inhibitor [PloS Biology, 6(10), 2237-2247 (2008)], miRNAs such as miR-291-3p, miR-294, and miR-295 [R.L. Judson et al., Nat. Biotech., 27:459-461 (2009)], and the like can be used.

Examples of culture media for iPS cell induction include (1) a DMEM, DMEM/F12 or DME medium containing 10 to 15% FBS (these media can further contain LIF, penicillin/streptomycin, puromycin, L-glutamine, non-essential amino acids, β-mercaptoethanol and the like), (2) an ES cell culture medium containing bFGF or SCF, for example, a mouse ES cell culture medium (e.g., TX-WES medium, Thromb-X NV) or a primate ES cell culture medium [e.g., primate (human and monkey) ES cell culture medium, ReproCELL, Kyoto, Japan], and the like.

In a culture method, for example, somatic cells and a nuclear reprogramming substance (DNA or protein) are brought into contact with each other on a DMEM or DMEM/F12 medium containing 10% FBS and cultured at 37°C in the presence of 5% CO₂ for about 4 to about 7 days, after which the cells are reseeded onto feeder cells (e.g., STO cells, SNL cells and other cells, previously treated with mitomycin C), and again cultured using a bFGF-containing primate ES cell culture medium, starting about 10 days after contact of the somatic cells and the nuclear reprogramming substance, whereby iPS-like colonies can be produced in about 30 to about 45 days or more after the contact. To increase the efficiency of iPS cell induction, the somatic cells may be cultured under conditions involving a low oxygen concentration of 5-10%.

Alternatively, the cells may be cultured on feeder cells (e.g., STO cells, SNL cells and other cells, previously treated with mitomycin C), using a DMEM medium containing 10% FBS (this can further contain LIF, penicillin/streptomycin, puromycin, L-glutamine, non-essential amino acids, β-mercaptoethanol and the like), whereby ES-like colonies can be produced after about 25 to about 30 days or more.

During the period of cultivation, the medium is replaced with a fresh supply of the same medium once daily starting on day 2 of cultivation. Although the number of somatic cells used for nuclear reprogramming is not subject to limitations, it falls in the range of about 5x10³ to about 5x10⁶ cells per 100 cm² of culture dish.

When a drug resistance gene is used as a marker gene, cells that express the marker gene can be selected by cultivation using a medium containing the corresponding drug (selection medium). Cells that express the marker gene can be detected by making an observation using a fluorescence microscope for a fluorescent protein gene as the marker gene, by adding a luminescent substrate for a luminescent enzyme gene as the marker gene, and by adding a color developing substrate for a color developing enzyme gene as the marker gene.

Any cells, other than germ cells, of mammalian origin (e.g., humans, mice, monkeys, pigs, rats and the like) can be used as the "somatic cells" used in the present invention. Examples include keratinizing epithelial cells (e.g., keratinized epidermal cells), mucosal epithelial cells (e.g., epithelial cells of the superficial layer of tongue), exocrine gland epithelial cells (e.g., mammary gland cells), hormone-secreting cells (e.g., adrenomedullary cells), cells for metabolism or storage (e.g., liver cells), intimal epithelial cells constituting interfaces (e.g., type I alveolar cells), vascular endothelial cells), cells having cilia with transporting capability (e.g., airway epithelial cells), cells for extracellular matrix secretion (e.g., fibroblasts), constrictive cells (e.g., smooth muscle cells), cells of the blood and the immune system (e.g., T lymphocytes), sense-related cells (e.g., rod cells), autonomic neurons (e.g., cholinergic neurons), sustentacular cells of sensory organs and peripheral neurons (e.g., satellite cells), neurons and glia cells in the central nervous system (e.g., astroglia cells), pigment cells (e.g., retinal pigment epithelial cells), progenitor cells (tissue progenitor cells) thereof and the like. There is no limitation on the degree of cell differentiation, the age of the animal from which cells are collected and the like; even undifferentiated progenitor cells (including somatic stem cells) and finally differentiated mature cells can be used alike as sources of somatic cells in the present invention. Here, examples of undifferentiated progenitor cells include tissue stem cells (somatic stem cells) such as neural stem cells, hematopoietic stem cells, mesenchymal stem cells, and dental pulp stem cells.

The choice of mammalian individual from which somatic cells are collected is not particularly limited, but it is preferably a human. The somatic cells are collected from a patient with ALS (particularly, familial ALS) or a healthy person having a genetic polymorph that correlates with the disease. Here, genetic polymorphs include, but are not limited to, polymorphs with a mutation in the coding region of SOD1. Preferred mutations of SOD1 are mutations that do not cause the SOD activity of SOD1 to be lost (the activity may decrease, as far as the required superoxide elimination capacity is retained). Examples of such mutations include mutations in the 4th exon in the mRNA of SOD1 (308-505 in the mRNA), more specifically mutations wherein leucine at position 106 is converted, preferably mutations wherein the leucine is replaced with another amino acid, preferably valine.

### Method for inducing differentiation into neural stem cells

In the present disclosure, a neural stem cell refers to a cell capable of differentiating into a neuron, an astrocyte and an oligodendrocyte, and also capable of self-renewal.

The method of inducing the differentiation from the above-described iPS cells to neural stem cells is not particularly limited; useful methods include differentiation by high-density culture on a fibroblast feeder layer (JP-A-2008-201792), differentiation by co-cultivation with stromal cells (SDIA method) (e.g., WO2001/088100, WO/2003/042384), differentiation by suspended culture (SFEB method) (WO2005/123902) and a combination thereof.

Preferred iPS cells are cells induced from a somatic cell derived from a patient with amyotrophic lateral sclerosis. The cells used in the method of the invention have a mutation in SOD1. Here, the mutation of SOD1 is a mutation that causes amyotrophic lateral sclerosis, for example, a mutant in leucine at position 106, more specifically the SOD1 mutant shown by SEQ ID NO:1 or SEQ ID NO:2.

In the method of the invention, the desired differentiation is induced by forming neurospheres after an iPS cell is cultured in an optionally chosen medium in a coated culture dish.

Here, examples of coating agents include collagen, gelatin, poly-L-lysine, poly-D-lysine, fibronectin, laminin and combinations thereof, with preference given to a combination of poly-L-lysine and laminin.

The medium can be prepared by adding additives to any basal medium useful for animal cell culture. Such basal media include, for example, the Neurobasal medium, Neural Progenitor Basal medium, NS-A medium, BME medium, BGJb medium, CMRL 1066 medium, Glasgow MEM medium, Improved MEM Zinc Option medium, IMDM medium, Medium 199 medium, Eagle MEM medium, αMEM medium, DMEM medium, DMEM/F12 medium, Ham medium, RPMI 1640 medium, Fischer's medium, and mixed media thereof, with preference given to a mixture of the Neurobasal medium and DMEM/F12. Additives include serum, KSR(KO serum replacement), retinoic acid, BMP inhibitors, TGF-beta inhibitors, bFGF, EGF, HGF, LIF, amino acids, vitamins, interleukins, insulin, transferrin, heparin, heparan sulfate, collagen, fibronectin, progesterone, selenite, the B27-supplement, the N2-supplement, the ITS-supplement, and antibiotics. Nogin, Dorsomorphin or LDN913189 as the BMP inhibitor, SB43154 as the TGF-beta inhibitor, the amino acid glutamine, the B27-supplement and the N2-supplement are exemplified as preferred additives.

The iPS cell density at the start of cultivation can be set as appropriate to allow neural stem cells to be formed efficiently. The iPS cell density at the start of cultivation is not particularly limited, and is, for example, about 1x10³ to about 1x10⁶ cells/ml, preferably about 1x10⁴ to about 5x10⁵ cells/ml.

Other culturing conditions such as culturing temperature and CO₂ concentration can be set as appropriate. The culturing temperature is not particularly limited, and is, for example, about 30 to 40°C, preferably about 37°C. The CO₂ concentration is, for example, about 1 to 10%, preferably about 5%.

Neurospheres can be formed using one of the above-described basal media and additives. A preferred medium is a mixture of the Neurobasal medium and DMEM/F12. Serum, Nogin, Dorsomorphin or LDN913189 as the BMP inhibitor, SB43154 as the TGF-beta inhibitor, bFGF, EGF, heparin, the B27-supplement and the N2-supplement are exemplified as preferred additives.

The cell density at the start of neurosphere formation can be set as appropriate to allow neurospheres to be formed efficiently. The cell density at the start of cultivation is not particularly limited, and is, for example, about 1x10⁴ to about 5x10⁶ cells/ml, preferably about 5x10⁵ to about 2x10⁶ cells/ml.

In forming neurospheres, they permit passage when they have grown to an appropriate size. Although the number of days of each passage is not subject to limitations, the interval is usually 5, 7, 10, 14, 15, 21, 28, 30 or 45 days, more preferably 30 days. In the passage, the cells may not be dissociated completely, and dissociation may be achieved using a mechanical means or a dissociation solution having both protease activity and collagenase activity. Passages might be done once, twice, three-times or four-times, but is not particularly limited.

In forming neurospheres, the culture vessel is preferably non-cell-adhesive or low-cell-adhesive. A useful non-cell-adhesive culture vessel is a culture vessel whose surface is not treated to artificially increase adhesion of cells thereto (e.g., coating with extracellular matrix and the like), or a culture vessel treated to artificially suppress adhesion of cells thereto [e.g., coating with polyhydroxyethyl methacrylate (poly-HEMA)]. The low-cell-adhesive culture vessel is coated with LIPIDURE (NOF CORPORAION).

Other culturing conditions such as temperature and CO₂ concentration at the time of neurosphere formation can be set as appropriate. The culturing temperature is not particularly limited, and is, for example, about 30 to 40°C, preferably about 37°C. The CO₂ concentration is, for example, about 1 to 10%, preferably about 5%.

The neural stem cells thus induced can be identified using expression markers of primitive neuroectoderms and neural stem cells, such as N-CAM, polysialylated N-CAM, A2B5, intermediate filament proteins like nestin and vimentin, and the transcriptional factor Pax-6. Preferably, the neural stem cells are identified by the expression of nestin.

### Method for inducing differentiation into astrocytes

Astrocytes can be induced by dissociating the neural stem cells induced by the method described above, by an optionally chosen method, and culturing them in an optionally chosen medium in a coated culture dish.

This dissociation can be achieved using a mechanical means or a dissociation solution having both protease activity and collagenase activity (e.g., Accutase^{™} and Accumax^{™}).

Examples of coating agents include collagen, gelatin, poly-L-lysine, poly-D-lysine, fibronectin, laminin, and combinations thereof, with preference given to gelatin.

Media of different compositions can be used after elapse of an optionally chosen time. Each medium cay be prepared by adding additives to a basal medium. Here, any basal medium useful for animal cell culture can be used. Examples include the Neurobasal medium, Neural Progenitor Basal medium, NS-A medium, BME medium, BGJb medium, CMRL 1066 medium, Glasgow MEM medium, Improved MEM Zinc Option medium, IMDM medium, Medium 199 medium, Eagle MEM medium, αMEM medium, DMEM medium, DMEM/F12 medium, Ham medium, RPMI 1640 medium, Fischer's medium, and mixed media thereof, with preference given to a mixture of the Neurobasal medium and DMEM/F12, or the DMEM medium. Additives here include serum, retinoic acid, BMP, bFGF, EGF, HGF, LIF, ciliary neurotrophic factor (CNTF), amino acids, vitamins, interleukins, insulin, transferrin, heparin, heparan sulfate, collagen, fibronectin, progesterone, selenite, the B27-supplement, the N2-supplement, the ITS-supplement, and antibiotics, with preference given to serum, BMP, LIF, the B27-supplement, and the N2-supplement. Here, the serum concentration is preferably 0.5% or more, more preferably 1% or more. The BMP is BMP-2 or BMP-4. The medium used is a DMEM/F12 mixed medium supplemented with serum, BMP-4, LIF, the B27-supplement and the N2-supplement (N2B27), or a DMEM medium supplemented with serum. Here, it is desirable that the neural stem cells be first cultured using an N2B27 medium containing BMP-4 and LIF, then cultured using the DMEM medium instead.

The neural stem cell density at the start of cultivation can be set as appropriate to allow astrocytes to be formed efficiently. The neural stem cell density at the start of cultivation is not particularly limited, and is, for example, about 1x10³ to about 1x10⁶ cells/ml, preferably about 1x10⁴ to about 5x10⁵ cells/ml.

Other culturing conditions such as culturing temperature and CO₂ concentration can be set as appropriate. The culturing temperature is not particularly limited, and is, for example, about 30 to 40°C, preferably about 37°C. The CO₂ concentration is, for example, about 1 to 10%, preferably about 5%.

The astrocytes thus induced can be identified by the expression of GFAP, which, however, is not to be construed as limiting the scope of the present invention.

### Screening method for prophylactic and therapeutic drug for amyotrophic lateral sclerosis

The present invention provides a method of screening for a candidate substance for a prophylactic and therapeutic drug for amyotrophic lateral sclerosis with the SOD1 expression suppressing action in astrocytes as an index, wherein astrocytes derived from iPS cell obtained as described above and a test substance are brought into contact with each other. In the present invention, preferred astrocytes are cells induced from an iPS cell derived from a patient with amyotrophic lateral sclerosis, wherein the cells have a mutation in SOD1. Here, the mutation in SOD1 is a mutation that causes amyotrophic lateral sclerosis, and, for example, a mutant of leucine at position 106, specifically the SOD1 mutant shown by SEQ ID NO:1 or SEQ ID NO:2 can be mentioned.

The test substance in the present invention may be any commonly known compound or a novel compound; such substances include, for example, nucleic acids, glucides, lipids, proteins, peptides, organic low molecular compounds, compound libraries prepared using combinatorial chemistry technology, random peptide libraries prepared by solid phase synthesis or the phage display method, or naturally occurring ingredients derived from microorganisms, animals, plants, marine organisms and the like, and the like. value of SOD1 detected in astrocytes not brought into contact with a test substance and the value of SOD1 detected in the same astrocytes brought into contact with the test substance are compared. If the detected value of SOD1 in the cells in contact with the test substance is lower, the test substance is selected as a candidate substance for a prophylactic and therapeutic drug for amyotrophic lateral sclerosis.

In detecting SOD1, the amount of the mRNA or protein of SOD1 expressed is measured, and this measured amount of expression can be used as a detected value. The amounts of the mRNA and protein of SOD1 expressed can be measured using a method known per se. For example, the amount of the mRNA expressed can be measured by a method such as Northern blotting or RT-PCR, and the amount of the protein expressed can be measured by an immunological method such as ELISA or Western blotting.

The test substance thus screened for can be used as a prophylactic and therapeutic drug for amyotrophic lateral sclerosis.

### Prophylactic and therapeutic agent for amyotrophic lateral sclerosis

The present description discloses a prophylactic and therapeutic agent for amyotrophic lateral sclerosis (hereinafter, also referred to as a prophylactic and/or therapeutic agent for ALS) with an HMG-CoA reductase inhibitor as an active ingredient.

In the present disclosure, it is desirable that the amyotrophic lateral sclerosis to be treated be familial amyotrophic lateral sclerosis. In a more preferred embodiment, the subject disease is familial amyotrophic lateral sclerosis having a mutation of SOD1, for example, cases wherein the mutation of the SOD1 gene does not cause the SOD activity of the gene product to be lost. As examples of mutations that do not cause the SOD activity of the SOD1 gene product to be lost, mutations in the 4th exon in the mRNA of SOD1 (308-505 in the mRNA), specifically mutations wherein leucine at position 106 is converted, preferably mutations wherein the leucine is substituted by another amino acid, preferably by valine, can be mentioned.

The HMG-CoA reductase inhibitor as an active ingredient of the prophylactic and/or therapeutic agent for ALS in the present disclosure may be any of natural substances of microbial origin, semi-synthetic substances derived therefrom, and fully synthetic compounds, which are statin compounds exemplified by (+)-(3R,5R)-3,5-dihydroxy-7-[(1S,2S,6S,8S,8aR)-6-hydroxy-2-methyl-8-[(S)-2-methylbutyryloxy]-1,2,6,7,8,8a-hexahydro-1-naphthyl]heptanoic acid [pravastatin, see JP-A-SHO-57-2240 (USP4346227)], (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl (S)-2-methyl butyrate [lovastatin, see JP-A-SHO-57-163374 (USP4231938)], (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl 2,2-dimethyl butyrate [simvastatin, see JP-A-SHO-56-122375 (USP4444784)], (±)(3R*,5S*,6E)-7-[3-(4-fluorophenyl)-1-(1-methylethyl)-1H-indol-2-yl]-3,5-dihydroxy-6-heptenoic acid [fluvastatin, see JP-T-SHO-60-500015 (USP4739073)], (3R,5S)-7-[2-(4-fluorophenyl)-5-(1-methylethyl)-3-phenyl-4-phenylaminocarbonyl-1H-pyrrol-1-yl]-3,5-dihydroxyheptanoic acid [atorvastatin, see JP-A-HEI-3-58967 (USP5273995)], (+)-(3R,5S)-7-[4-(4-fluorophenyl)-6-isopropyl-2-(N-methyl-N-methanesulfonylamino)pyrimidyn-5-yl]-3,5-dihydroxy-6(E)-heptenoic acid [rosuvastatin, see JP-A-HEI-5-178841 (USP5260440)] and (E)-3,5-dihydroxy-7-[4'-(4"-fluorophenyl)-2'-cyclopropylquinolin-3'-yl]-6-heptenoic acid [pitavastatin, see JP-A-HEI-1-279866 (USP5854259 and USP5856336)].

A preferred HMG-CoA reductase inhibitor is atorvastatin.

In the HMG-CoA reductase inhibitor as an active ingredient of the prophylactic and/or therapeutic agent for ALS of the present disclosure, pravastatin, lovastatin, simvastatin, fluvastatin, atorvastatin, rosuvastatin or pitavastatin includes a lactone ring isomers thereof and pharmacologically acceptable salts thereof (suitably a sodium salt or calcium salt and the like).

The present invention is hereinafter described in further detail by means of the following Example, to which, however, the invention is never limited.

### Example

### Fibroblasts derived from a patient with amyotrophic lateral sclerosis (ALS)

A 4-mm skin biopsy was cultured for 3 weeks, and the resulting cells in culture were used as the fibroblasts derived from an ALS patient.

### Induction of iPS cells

Human cDNAs for KLF4, Sox2, Oct3/4 and c-Myc were transferred to the above-described fibroblasts using a retrovirus, as described by Takahashi K et al. in Cell 131(5), 861, 2007. On day 6 after the gene transfer, the fibroblasts were transferred onto SNL feeder cells. The following day, the medium was replaced with a primate ES cell culture broth supplemented with 4 ng/ml of bFGF (Wako). The medium was exchanged every two days; 30 days after the gene transfer, colonies were picked up. An analysis of the sequence of the SOD1 of the iPS cell established here revealed the presence of a mutation for substitution of the leucine at position 106 (amino acids were numbered for a protein deprived of the first methionine) by valine.

### Formation of neurospheres

Neurospheres were formed by a slightly modified version of the method described by Wada T et al. in PLoS ONE 4(8), e6722, 2009. Specifically, iPS cells were divided into small masses, and cultured in a dish coated with poly-L-lysine/laminin (PLL/LM) (Sigma-Aldrich) using the N2B27 medium (Gibco), which is prepared by adding 1% N2, 2% B27 and 200 µM glutamine to a culture broth of a 1:1 mixture of DMEM/F12 and the Neurobasal medium A. Furthermore, 100 ng/ml of Noggin was added to this medium. At 3-day intervals, the medium was replaced with a medium containing 100 ng/ml of Noggin; after 10 days of cultivation, the cells were passaged to a PLL/LM-coated dish. Subsequently, the medium was replaced with a medium containing 100 ng/ml of Noggin every other day. 7 days after the passage, the cells were seeded to a 2-hydroxyethylmetacrylate (HEMA)-coated dish at a cell density of 1,000,000 cells/ml and neurospheres were formed. The medium used was an N2B27 medium supplemented with 20 ng/ml of EGF (R&D Systems), 20 ng/ml of bFGF and 50 ng/ml of heparin (Sigma-Aldrich). Passage was performed with pipetting at 30-day intervals; 1 ml of a fresh medium was added at 7-day intervals. All these culturing operations were performed by incubation at 37°C, 5% CO₂, in a moisturized atmosphere.

### Induction of differentiation of astrocytes

Quaternary neurospheres were separated using Accutase, and seeded into an N2B27 medium containing 1% FBS (Japan Bio Serum), 10 ng/ml bone morphogenetic protein-4 (BMP-4) (R&D Systems) and 10 ng/ml leukemia inhibitory factor (LIF) (Alomone Labs) at a density of 50,000 cells/ml on a gelatin-coated dish. The medium was replaced with a fresh supply at 2-day intervals; 1 week later, the medium was replaced with a DMEM supplemented with 10% FBS and 1% penicillin/streptomycin. By this method, GFAP-positive cells were obtained, confirming the induction of differentiation into astrocytes.

### Analysis of SOD1 in astrocytes

The iPS cell-derived astrocytes were seeded to a 6-well plate at a density of 250,000 cells/well. After a vehicle [0.5% dimethylsulfoxide (DMSO)], cycloheximide at a final concentration of 5 µg/ml, and Atorvastatin Calcium Salt (Toronto Research Chemicals Inc.) at various final concentrations (5 nM, 50 nM, 500 nM and 5 µM) were added, the plate was incubated for 48 hours. Subsequently, the cells were recovered, and lysed using a 20 mM Hepes, pH 7.4, containing 1% triton X-100, 10% glycerol, 5 mM EDTA, 120 mM NaCl and protease inhibitor cocktail (Complete; Roche), on ice for 30 minutes. The cell lysate was centrifuged at 15,000 rpm, 4°C for 30 minutes, and the supernatant was recovered. Western blotting was performed using this cytolysis supernatant. Specifically, 20 µg aliquot of the protein was separated by SDS-PAGE (4-12% polyacrylamide gels), transferred to a PVDF membrane, and incubated with an anti-SOD1 antibody (1:2000 dilution) (Stressgen Company) or an anti-β-actin antibody (1:5000 dilution) (Sigma-Aldrich Company). After the incubation, the amount expressed was detected by ECL (Enhanced ChemiLuminescence) using an HRP-linked anti-rabbit IgG antibody (1:5000; GE healthcare) and an HRP-linked anti-mouse IgG antibody (1:5000; GE healthcare). As a result, at all concentrations of Atorvastatin Calcium Salt, a reduction in the expression of SOD1 was observed (Fig. 1). Meanwhile, when the vehicle DMSO alone was added to the human iPS cell 253G4 described by Nakagawa M et al. in Nat. Biotechnol. 26: 101-6, 2008 and the above-described SOD1 mutation iPS cell, the amount of SOD1 expressed did not change. Judging from these facts, combined with many findings concerning the effects of astrocytes having a mutation in SOD1 on motor neuron damage [Yamanaka K et al., Nat. Neurosci. 11(3): 251, 2008 and Nagai et al., Nat. Neurosci. 10: 615, 2007], it is suggested that atorvastatin may be useful in preventing or treating ALS.

## Claims

1. An *ex vivo* method of screening for a prophylactic and therapeutic drug for amyotrophic lateral sclerosis (ALS), comprising the steps of:
(1) differentiating an astrocyte from an induced pluripotent stem (iPS) cell having a mutation of Cu/Zn superoxide dismutase (SOD1) that correlates with ALS by (a) forming a neurosphere from the iPS cell, and (b) culturing the neurosphere in a medium in a coated culture dish,
(2) bringing into contact with each other the astrocyte and a test compound,
(3) measuring the amount of SOD1 expressed in the astrocyte, and
(4) selecting a test compound that reduces the amount of SOD1 expressed, compared with a control not brought into contact with the test compound.

2. The screening method according to claim 1, wherein the iPS cell is an iPS cell having a mutation from leucine to valine at position 106 of SOD1.

3. The screening method according to claim 2, wherein the medium for culturing the neurosphere contains a leukemia inhibitory factor (LIF) and a bone morphogenetic protein (BMP).

## Patentansprüche

1. Ex-vivo-Verfahren zum Screening nach einem prophylaktischen und therapeutischen Wirkstoff gegen amyotrophe Lateralsklerose (ALS), umfassend die Schritte:
(1) Differenzieren eines Astrocyten ausgehend von einer induzierten pluripotenten Stammzelle (iPS), die eine Mutation der Cu/Zn-Superoxiddismutase (SOD1) aufweist, welche mit ALS korreliert, durch (a) Bilden einer Neurosphäre aus der iPS-Zelle und (b) Kultivieren der Neurosphäre in einem Medium in einer beschichteten Kulturschale;
(2) In-Kontakt-Bringen des Astrocyten mit einer Testverbindung;
(3) Messen der Menge des in dem Astrocyten exprimierten SOD1; und
(4) Auswählen einer Testverbindung, die die Menge des exprimierten SOD1 im Vergleich zu einer Kontrolle, die nicht mit der Testverbindung in Kontakt gebracht wurde, reduziert.

2. Screeningverfahren gemäß Anspruch 1, wobei die iPS-Zelle eine iPS-Zelle ist, die eine Mutation von Leucin zu Valin auf der Position 106 von SOD1 aufweist.

3. Screeningverfahren gemäß Anspruch 2, wobei das Medium zum Kultivieren der Neurosphäre einen leukämiehemmenden Faktor (LIF) und ein knochenmorphogenetisches Protein (BMP) enthält.

## Revendications

1. Méthode *ex vivo* de criblage d'un médicament prophylactique et thérapeutique pour la sclérose latérale amyotrophique (SLA), comprenant les étapes suivantes :
(1) la différenciation d'un astrocyte à partir d'une cellule souche pluripotente induite (iPS) ayant une mutation de Cu/Zn superoxyde dismutase (SOD1) qui est corrélée à la SLA par (a) la formation d'une neurosphère à partir de la cellule iPS, et (b) la culture de la neurosphère dans un milieu dans un récipient de culture revêtu,
(2) la mise en contact de l'astrocyte et d'un composé à tester,
(3) la mesure de la quantité de SOD1 exprimée dans l'astrocyte, et
(4) la sélection d'un composé à tester qui réduit la quantité de SOD1 exprimée par rapport à un témoin qui n'a pas été mis en contact avec le composé à tester.

2. Méthode de criblage selon la revendication 1, dans laquelle la cellule iPS est une cellule iPS ayant une mutation d'une leucine en valine en position 106 de la SOD1.

3. Méthode de criblage selon la revendication 2, dans laquelle le milieu de culture de la neurosphère contient un facteur inhibant les cellules leucémiques (LIF) et une protéine osseuse morphogénétique (BMP).
